Europäisches Patentamt

European Patent Office

Office européen des brevets

(11) Publication number: **0 324 279 B1**

# EUROPEAN PATENT SPECIFICATION

45 Date of publication of patent specification: **30.08.95**  51 Int. Cl.⁶: **A61B 5/103**, A61B 17/56

(21) Application number: **88312388.7**

(22) Date of filing: **29.12.88**

54 **System for measuring fracture stiffness.**

(30) Priority: **29.12.87 GB 8730257**

Date of publication of application:
**19.07.89 Bulletin 89/29**

45 Publication of the grant of the patent:
**30.08.95 Bulletin 95/35**

Designated Contracting States:
**CH DE FR GB IT LI SE**

56 References cited:

ENGINEERING IN MEDICINE vol. 16, no. 4,
October 1987, pages 229-232, London, GB;
J.L. CUNNINGHAM et al.: "The measurement
of stiffness of fractures treated with external
fixation".

ENGINEERING IN MEDICINE vol. 11, no. 3,
July 1982, pages 123,124, Whitstable, GB;
W.V. JAMES et al.: "A clinical elec-
trogoniometry system".

JOURNAL A vol. 24, no. 3, July 1983, pages
139-153, Antwerpen, Belgium; R. VAN
CAUTEREN:"(Unusual) sensors in (unusual)
mechanical applications".

Proprietor: **ORTHOFIX S.r.l.**
**9, Via Delle Nazioni (Z.I.)**
**I-37012 Bussolengo (VR) (IT)**

Inventor: **Richardson, James Bruce, Prof.**
**Westminster House,**
**Old Chirk Road**
**Gobowen, Oswestry,**
**Shropshire SY11 3LW (GB)**
Inventor: **Roberts, Andrew, Dr.**
**The Robert Jones & Agnes Hunt Ortho-**
**paedic Hospital**
**Oswestry,**
**Shropshire SY10 7AG (GB)**
Inventor: **Shah, Khalid, Dr.**
**Flat 8/1 Dadabhoy Centre,**
**Opposite Aisha Bawany**
**Academy**
**Karachi 75500 (PK)**

Representative: **Milhench, Howard Leslie et al**
**R.G.C. Jenkins & Co.**
**26 Caxton Street**
**London SW1H 0RJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a system for measuring fracture stiffness, which is indicative of fracture healing. The invention is particularly appliable to fractures of the bones of limbs.

The chief function of the skeleton is to support, ie structural, and this function is lost when a bone fails by fracture. Fracture healing returns this function, and therefore a mechanical measure may be appropriately employed to measure healing. The gradual change in tissue during healing can be characterised by its stiffness, changing from a Young's modulus of elasticity expressed as 0.005 Kp/mm2 for granulation tissue, as found in the new bone of a bridging callus, to 2000 Kp/mm2 for mature bone, representing a 400,000-fold change.

A fracture stiffness measurement as a functional measure of healing helps in two areas: comparisions between patients, and monitoring progress of an individual patient. The principle demands on the measuring system will be for accuracy of the stiffness measurement, to form a measure as a known, trusted and accurate standard or series of standards in the matter of comparison, and for precision of the stiffness measurement, to assess an individual patient's progress wherein the changes, especially on a day to day basis, may be very slight.

It is known that a fracture stiffness measurement is a good indicator for fracture healing and it is known that the degree of angular deflection of the healing tissue bridging the fracture is a good measurement of the stiffness, but conventional and previous methods and apparatus have been unsuccessful at producing an accurate, precise system of measurement, particularly when the fracture is not subjected to invasive apparatus such as bone screws, and which system is also relatively painless and safe.

Prior systems include the use of temporary percutanous bone screws inserted under anaesthesia, the movement of which under the application of a load is a measurement of fracture stiffness. This system is somewhat extreme and has the disadvantage of allowing little in the way of an assessment of the progress of healing, as patients do not tolerate frequently repeated measurements.

A radiographic prior art method has high errors for the precise measurements required for individual patient progress and it can not be improved by the simple procedure of repeating the tests as this would involve excessive exposure to X-rays.

An inaccurate but simple method of measuring fracture stiffness is to use a dial gauge micrometer to determine the amount of bending during application of a fixed torque to patients with external fixation. As expected the deflection fell with increasing stiffness and, by calculation these measurements have been expressed as a stiffness to allow comparison for other workers. However, the difficulties of obtaining a precise and accurate reading from such micrometers are known and the system is also not applicable to non-invasive apparatus.

Other systems have used strain gauges bonded to a patient's fixator. Calculation of the fixator's stiffness is required to estimate the fracture stiffness. However, these systems do have the advantage of repeatability an precision for an individual's progress.

Such a system is described in Engineering in Medicine; Vol 16, No 4, October 1987, Pages 229-232; J L Cunningham et al. "The Measurement of Stiffness of Fractures Treated with External Fixation". In the system described in this document the fracture stiffness is calculated from the known fixator stiffness where the fixator is arranged parallel to the fractured bone. The fracture stiffness is calculated from the known or measured parameters of the bending load applied, the bending moment measured in the centre of the fixator column, the average bone screw length joining the fixator to the fractured bone, and the fixator stiffness.

As abovementioned, there is a need for an objective stiffness measurement of reasonable accuracy to allow safer decisions as to when to remove fixators, external or invasive, and allow independent weight bearing. The commonest major complication, noted after use of certain prior art systems, is refracture after removal of the fixator or a significant loss of alignment, particularly in those limbs allowed free of support at a lower level of stiffness. Yet the fixators must not be in place too long, as this leads to further complications, such as stiffness of the joints.

It is highly desirable, and not unreasonable to anticipate, the use in future of one standard measurement to calculate the safe levels to allow independent weight-bearing; if a patient were heavy, or a fracture angulated, then higher levels of stiffness might be appropriate, but for purposes of a comparative trial, or to allow, for instance, international comparisons, one level of healing stiffness is appropriate.

Clearly also, another requirement of any fracture stiffness measurement apparatus is one that does not directly measure the strength of the bone (by weighting for example) as to do so would result in re-fracturing.

It is an object of the present invention to obviate or mitigate the abovementioned disadvantages.

According to the present invention there is provided an apparatus as defined in claim 11 and a method according to claims 1-10.

The spacers in the case of invasive fixators are in the form of arcuate clamp plates clampable to the invasive fixators, such as bone screws or bone pins, and of a form to position the goniometer substantially parallel to the anterior long axis of the bone, irrespective of the position of the fixators. These spacers bring the goniometer into the same vertical plane as the fracture and thus prevent errors due to rotation at the fracture.

The spacers in the case of non-invasive fixators, are in the form of bridges moulded to fit the limb having the fractured bone.

The bridges depend on good contact with the skin that overlies the generally anterior aspect of limb and employ a system of strapping having a three-point fixation including an elastic strap. A clamping system is used for fractures near the ankle. This clamp applies compression to the malleoli and thus achieves good stability.

The bridges may be connected to a cast or stocking or brace to reduce the skin movement effect on the measurement.

The means to measure the signal of the goniometer is connected with one spacer and includes an amplifier; said means may also be interconnected with said load measuring means and said calculating means.

There are included means for to compare the measurement with a reference measurement, either a reference measurement of the same fracture at an earlier date or measurement of the stiffness of the intact limb.

Preferably, there are means to record the reference measurement, particularly the reference measurement from apparatus applied to an invasive fixator system, and means to objectively compare such a measurement with a measurement obtained from apparatus applied to non-invasive or invasive fixator systems.

Embodiments of the present invention will now be described, by way of example, with reference to the accompanying drawings, in which:

Fig. 1 is a side elevation of one embodiment of apparatus for measuring fracture stiffness according to the present invention, in use on a tibial fracture;

Fig. 2 is a sectional view on x-x' of Fig. 1;

Fig. 3 is a schematic illustration of the angular deflection measured by the system of Figs 1 and 2;

Fig. 4 is a perspective view from one side of the goniometer of Figs 1 and 2;

Fig. 5 is a cross-sectional view of the goniometer of Fig. 4;

Fig. 6 is a schematic illustration of a second embodiment of apparatus according to the present invention, in use on a tibial fracture; and,

Fig 7 is a schematic illustration of an embodiment of a clamp of apparatus according to the present invention, in use on an tibial fracture near the ankle.

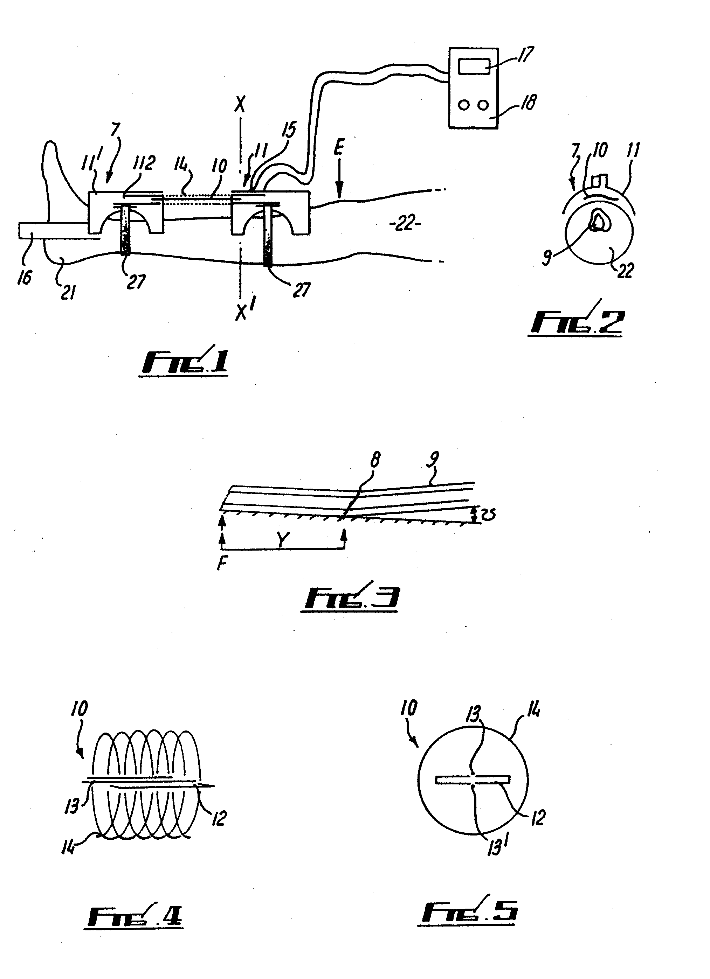

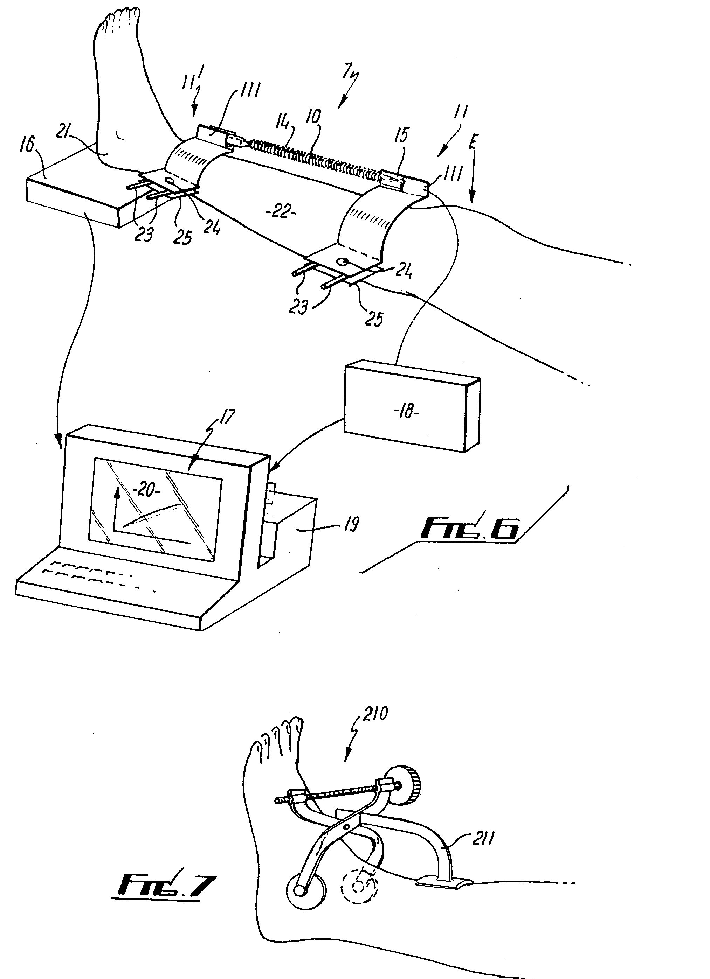

Referring to Figs 1 to 7, there is shown apparatus 7 of the present invention for measuring fracture stiffness, fracture stiffness being indicative of the healing of a fracture 8 and being calculated by measuring the angular deflection of the bone 9 on the fracture site 7.

The apparatus 7 comprises a goniometer 10 which is positionable to run vertically above the anterior long axis of the bone 9 having the fracture 8. It is positionable by means of spacers 11, with one end of the goniometer 10 attached to a first spacer 11 placed axially to one side of the fracture 8 and the other end attached to a second spacer 11' placed axially to the other side of the fracture 8.

A goniometer 10 is an instrument to measure angles. Previous prior art designs have limitations of sensitivity or are inaccurate due to changes in their length during flexion which alters their surface strain. A high gain goniometer has been designed on a polycarbonate or other suitable beam. Strain gauges are bonded to its full length on both surfaces to maintain accuracy by covering the area within which the point of rotation of the limb will probably lie. The spacers include a sliding element so that altering the length of the device does not of itself produce angulation.

The electrogoniometer 10 used in the present invention, although previously known, has only been used before in the assessment of joint function in patients and thus used generally at a range of over 90° flexion. Yet it is infinitely sensitive, is relatively stable to temperature changes and has very little resistance to movement. It is therefore most appropriate as a means to measure the very slight angular deflection of a fracture site on the application of a load, but until now it had not been realised for this application; for fracture stiffness measurements angulation is of 2° or less and, importantly, over such slight angulation the force applied to the transducer does not change significantly.

More specifically, the goniometer 10 used here comprises a thin skim 12 of steel with wires 13,13′, one along either side (see Figs 4 and 5) of the skim 12 and wires 13,13′ being wrapped in a protective coiled spring 14. The wires 13,13′act as a strain gauges, their output having a linear relationship with the subtended angle. If the load applied is known, the amount of bending can be measured, this angular deflection being representative of fracture stiffness and, hence, fracture healing.

The apparatus 7 therefore includes means 15 to measure the signal from the goniometer 10, these means being connected to one spacer 11.

The apparatus 7 also includes means 16 to measure the load applied and means 17 to calculate the angular deflection with reference to this load-applied measurement. The goniometer signal measuring means 15 includes an amplifier 18 which connects with the calculating means 17, which is in the form of a computer with print-out 19 and screen display 20.

The load measuring means 16 are in the form of bathroom scales, which in the case of a tibial fracture are placed distally under the heel 21 or attached to the heel-based spacer 11′ of a patient and the load is applied proximally to the fracture at E. The load is applied at right angles to the long axis of the bone 9 which is placed unsupported along which of its length such that the load may be applied to the anterior and downwardly, aided by the pull of gravity and limb's 22 weight.

On the application of a load, the fractured tibia 9 bends downwardly; the distance over which the goniometer 10 extends is thus decreased and the goniometer bows. The degrees of angular deflection ($\vartheta$) is calculated by knowing the distance (y) from the point load measured (F) to the fracture site 8 (see Fig. 6).

$$\text{Applied Torque} = F\, y$$

$$\textbf{Fracture Stiffness} \quad = \quad \frac{F\, y}{\vartheta}$$

The errors between measurement have a 95% confidence limit of +/-12%.

Comparison with AP X-ray results shows that the goniometer 10 provides accurate results.

In the embodiment of Figs 5 and 6, the fixators are invasive being in the form of bone screws 23. The spacers 11 are therefore in the form of arcuate clamps 111, clampable to the screws 23, by a screw-threaded tightening nut 24 and back plate 25 and being of a form to position the goniometer 10 substantially parallel to the antieror long axis of the bone 9.

The spacers 11 of the embodiment of Figs 1 and 2 are for use with a non-invasive fixator system. The spacers 11 are in the form of orthoplast bridges 112. As the soft tissue attachment of the goniometer is a potential source of error in the system 7, each bridge 112 is developed with good contact with the skin that overlies the anterior aspect of the tibia 9, and a system of strapping 2 that uses a 3-point fixation, including an elastic strap 27.

In the embodiment of Fig 7, a clamping system 210 is used. This system is particularly useful for fractures near the ankle wherein the normal bridge would have insufficient tibia to attach to. The clamp 211 applies compression to the malleoli and this achieves good stability.

This invention allows an objective measure to be formulated to allow comparison of patients treated conservatively against those with external fixation; indeed, the former serves as the standard by which the latter are tested.

The apparatus 7 is also beneficial in that it is non-invasive, painless and not stressful for the patients, as it requires only a few degrees of angulation.

With such apparatus the precise measurement of an individual bone may be taken and this allows monitoring of the progress of healing for a specific patient. Even small changes in stiffness can be considered significant and can be studied with a view to the management of aspects of treatment, such as the rate of return to full weight-bearing. The clincian needs such information, as removal of splintage or fixators at too early a stage can lead to refracture or loss of alignment yet if allowed to remain for too long the liklihood increases of having further complications such as increased stiffness.

Once an objective standard is achieved one patient may be compared to another and this need not be limited to one type of treatment, so allowing comparison of different types of treatment.

An objective measurement will indicate complications; for example if low levels of stiffness persist, compared to a normal progress of individuals with a similar injury, operative intervention may be indicated. Indeed, an objectively defined healing time can be compared to clinical parameters of healing, and used to analyse the many variables that affect the progress of fracture healing in patients.

An objective standard will help predict the time to healing the certainty of prediction increasing as the stiffness rises. Such prediction will be useful on the individual basis as well as for comparison.

The use of fracture stiffness measurement also allows an objective testing of the effect of passive cyclic micromovement applied in the early weeks following injury.

The progress of fracture healing when there is only external fixators has previously been particularly difficult to assess. The objective stiffness measurement allows a safer decision of when to remove the tixation and allow independent weight bearing.

There is a particular need to compare the use of functional fixators with functional bracing for tibial fractures, particularly as the proposed indications for each method of management increase and overlap considerably in tibial fracture management. The present stiffness measuring system as an objective and sensitive measuring system and being applicable to invasive and non-invasive fixators, provides some of the answers regarding which method of treatment is appropriate in cases of tibial or other fractures.

The overall accuracy of the apparatus is improved by taking the mean of several repeated tests, and rapid feedback from a test to aid in the indentification of errors. The means for carrying out such comparisons means includes software for a portable computer that allows a visual display of output and calculates the fracture stiffness and predicts the time at which the individual fracture will heal. The software communicates with a database that holds data on a series of patients to allow comparison and assessment of progress.

Further accuracy may be obtained by the use of digitised load transducer in addition to a digitised output to the goniometer.

**Claims**

1. A method of measuring the stiffness of a bone fracture to obtain an indication of the progress of fracture healing, said method comprising:

affixing first and second mounting elements (11, 11') with respect to the fractured bone on opposite sides of the fracture site;

supporting a member between said first and second mounting elements;

loading the fracture site by application of a force thereto; and

monitoring the condition of said member under such loading;

the method being characterized in that:

said member supported between said first and second mounting elements comprises a goniometer (10) extending generally coplanar with the bone axis, said goniometer providing very little resistance to movement and there being no other means connecting together the opposite sides of the bone on either side of the fracture site, and said force being applied in the plane of the goniometer and the bone axis and in a direction transverse to the bone axis.

2. A method as claimed in claim 1 wherein the fracture is in an elongate bone of a limb and said first and second mounting elements comprise non-invasive bridges (112) shaped to fit over the limb on either side of the fracture site, the bridges being firmly affixed to the limb.

3. A method as claimed in claim 2 wherein the fracture is in a lower leg bone near to the ankle and the bridge that is near to the ankle is formed with clamping means (210) which are clamped to the ankle to fix the position of the respective bridge.

4. A method as claimed in claim 1 wherein the fracture is in an elongate bone of a limb and said first and second mounting elements (11,11') comprise clamps (111) which are clamped to invasive fixators (23) secured to the fractured bone on either side of the fracture site.

5. A method as claimed in any of the preceding claims wherein the fracture site is loaded by placement of a load cell (16) distally of the fracture site and applying a force to the fractured bone proximally of the fracture site.

6. A method as claimed in any of the preceding claims wherein the loading of the fracture site is effected so as to produce angulation ($\vartheta$) of the fractured bone of 2° or less.

7. A method as claimed in any of the preceding claims wherein the goniometer (10) is an electrogoniometer comprising an elongate thin skim (12) having strain gauge means (12,13,14) secured to at least one of its opposed surfaces whereby there can be derived from the electrogoniometer a signal representative of the angulation of the fractured limb when the fracture site is loaded.

8. A method as claimed in claim 7 wherein the elongate thin skim (12) comprises steel and the strain gauge means comprises wires (13,13') extending longitudinally with respect to the elongate skim (12).

9. A method as claimed in any of the preceding claims wherein an output signal is derived from the goniometer (10) which is indicative of the bone angulation under load, a signal indicative of the applied force is also provided, and a processor (17) receives said signals, and derives a fracture stiffness measurement as a function thereof.

10. An apparatus for use in carrying out the method of any of the preceding claims, said apparatus comprising:

a goniometer (10) of a kind providing very little reistance to movement;

first and second mounting elements (11,11') adapted for supporting said goniometer in coplanar relationship with the axis (9) of the fractured bone, said mounting elements being adapted to be affixed with respect to the fractured bone on opposite sides of the fracture site;

means (16) for measuring the loading of the fracture site produced by application of a force thereto in the plane of the goniometer (10) and the bone axis (9) and in a direction transverse to the bone axis (9); and

a processor (17) for receiving from the goniometer (10) a signal indicative of the angulation of the bone fracture site under load and for receiving from said loading measuring means a signal indicative of the load applied to the fracture site to produce such angulation, said processor (17) being arranged to derive from said angulation and loading signals a measure of the stiffness of the fracture site.

**Patentansprüche**

1. Verfahren zur Messung der Steifigkeit eines Knochenbruches, um somit Angaben über den Fortschritt der Bruchheilung zu erlangen, wobei dieses Verfahren umfaßt:

Anordnen von ersten und zweiten Befestigungselementen (11, 11') an dem gebrochenen Knochen an einandergegenüberliegenden Seiten des eigentlichen Bruches;

Anordnen eines Bauteiles zwischen dem ersten und zweiten Befestigungselement;

Belasten des Bruchbereiches durch Aufbringen einer Kraft und

Überwachen des Verhaltens des Bauteiles unter der Belastung,

dadurch gekennzeichnet, daß das zwischen dem ersten und zweiten Befestigungselement angeordnete Bauteil ein Goniometer (10) aufweist, das sich im wesentlichen coplanar mit der Knochenachse erstreckt, wobei dieses Goniometer einen sehr geringen Widerstand gegenüber Bewegungen schafft und keine weiteren Mittel vorgesehen sind, die die einandergegenüberliegenden Seiten des Knochens auf jeder Seite des Bruchbereiches verbinden und die genannte Kraft in der Ebene des Goniometers und der Knochenachse aufgebracht wird und in Richtung quer zur Knochenachse.

2. Verfahren nach Anspruch 1, wobei der Bruch in einem langgestreckten Knochen eines Körpergliedes vorliegt und die ersten und zweiten Befestigungsmittel nicht eindringende Brücken (112) aufweisen, die so ausgebildet sind, daß sie über das Körperglied auf beiden Seiten des Bruches greifen, wobei die Brücken fest mit dem Körperglied verbunden sind.

3. Verfahren nach Anspruch 2, wobei bei Vorliegen des Bruches im unteren Beinbereich nahe des Fußknöchels, die Brücke nahe des Fußknöchels mit Klemmitteln (210) ausgerüstet ist, die an dem Fußknöchel angeklemmt werden, um damit die Stellung der jeweiligen Brücke zu fixieren.

4. Verfahren nach Anspruch 1, wobei der Bruch in einem langgestreckten Knochen eines Körpergliedes vorliegt und die ersten und zweiten Befestigungsmittel (11, 11') Klammern (111) aufweisen, die an eindringenden Fixatoren (23) beiderseits des Bruchbereiches befestigt sind.

5. Verfahren nach einem der vorhergehenden Ansprüche, wobei der Bruchbereich durch eine Lastzelle (16) distal des Bruchbereiches belastet wird und eine Kraft proximal zum Bruchbereich auf den gebrochenen Knochen ausgeübt wird.

6. Verfahren nach einem der vorhergehenden Ansprüche, wobei die Belastung des Bruchbereiches dadurch erfolgt, daß eine Abwinkelung ($\vartheta$) des gebrochenen Knochens von 2° oder weniger erfolgt.

7. Verfahren nach einem der vorhergehenden Ansprüche, wobei das Goniometer (10) als Elektrogoniometer ausgebildet ist mit einem langgestreckten dünnen Ableiter (12) mit Meßmitteln (12, 13, 14), die wenigstens auf einer der gegenüberliegenden Oberflächen angeordnet sind, so daß dadurch von dem Elektrogoniometer ein Signal abgeleitet werden kann, das repräsentativ der Abwinkelung des gebrochenen Körpergliedes ist, wenn der Bruchbereich belastet wird.

8. Verfahren nach Anspruch 7, wobei der langgestreckte Ableiter (12) Stahl umfaßt und die Meßmittel Drähte (13, 13') umfassen, die sich in Längsrichtung gegenüber dem langgestreckten Ableiter (12) erstrecken.

9. Verfahren nach einem der vorhergehenden Ansprüche, wobei das Ausgangssignal vom Goniometer (10) erzeugt wird und indikativ für die Knochenabwinkelung unter Last ist, wobei weiterhin ein indikatives Signal für die aufgebrachte Kraft geschaffen wird und ein Prozessor (17) diese Signale aufnimmt und daraus eine Bruchsteifigkeitsmessung als Funktion davon ableitet.

10. Eine Vorrichtung zur Durchführung des Verfahrens nach einem der vorhergehenden Ansprüche, wobei diese Vorrichtung umfaßt:
   ein Goniometer (10) derart, daß es sehr kleine Widerstände gegenüber Bewegungen hat;
   erste und zweite Befestigungsmittel (11, 11'), die in der Lage sind, dieses Goniometer zu tragen, und zwar coplanar mit der Achse (9) des gebrochenen Knochens, wobei diese Befestigungsmittel in der Lage sind, beiderseits des gebrochenen Knochens angeordnet zu werden;
   Vorrichtungen (16) zur Messung der Last im Bruchbereich, die durch eine hier einwirkende Kraft erzeugt wird, und zwar in der Ebene des Goniometers (10) und der Knochenachse (9) und in einer Richtung quer zur Achse (9) des Knochens und
   einen Rechner (17) zur Aufnahme eines vom Goniometer (10) abgegebenen Signales, das indikativ für die Abwinkelung des Knochens im Bereich des Knochenbruches unter Last ist und von den Lastmeßeinrichtungen ein indikatives Signal erhält bezüglich der aufgebrachten Last in dem Bruchbereich, um eine solche Abwinkelung zu erreichen, wobei dieser Rechner (17) aufgebaut ist, um von dieser Abwinkelung und den Lastsignalen ein Maß der Steifigkeit des Bruchbereiches abzuleiten.

**Revendications**

1. Procédé de mesure de la rigidité d'une fracture d'un os pour obtenir une indication du progrès de la guérison de la fracture, ledit procédé comprenant :
   la fixation de premier et second éléments de montage (11, 11') de part et d'autre de l'emplacement de la fracture par rapport à l'os fracturé ;
   le support d'une pièce entre lesdits premier et second éléments de montage ;
   la mise en charge de l'emplacement de la fracture en appliquant une force à ce dernier ; et
   le contrôle de l'état de ladite pièce sous une telle mise en charge ;
   le procédé étant caractérisé en ce que :
   ladite pièce supportée entre lesdits premier et second éléments de montage comprend un goniomètre (10) s'étendant de manière globalement coplanaire à l'axe de l'os, ledit goniomètre offrant une très petite résistance au déplacement et aucun autre moyen n'existant pour relier ensemble les côtés opposés de l'os de part et d'autre de l'emplacement de la fracture, et ladite force étant appliquée dans le plan du goniomètre et de l'axe de l'os et dans une direction transversale à l'axe de l'os.

2. Procédé selon la revendication 1, dans lequel la fracture se situe dans un os allongé d'un membre et dans lequel lesdits premier et second éléments de montage sont constitués par des ponts non envahissants (112) formés pour s'ajuster au-dessus du membre de part et d'autre de l'emplacement de la fracture, les ponts étant solidement fixés au membre.

3. Procédé selon la revendication 2, dans lequel la fracture se situe dans un os inférieur de jambe, proche de la cheville, et le pont qui est proche de la cheville est formé par des moyens de serrage (210) qui

EP 0 324 279 B1

sont serrés à la cheville pour fixer la position du pont respectif.

4. Procédé selon la revendication 1, dans lequel la fracture se situe dans un os allongé d'un membre et dans lequel lesdits premier et second éléments de montage (11, 11') comprennent des attaches (111) qui sont serrées sur des dispositifs de fixation envahissants (23) solidement fixés à l'os fracturé de part et d'autre de l'emplacement de la fracture.

5. Procédé selon l'une quelconque des revendications précédentes, dans lequel l'emplacement de la fracture est chargé par placement d'une cellule de charge (16) à distance de l'emplacement de la fracture et par l'application d'une force à l'os fracturé à proximité de l'emplacement de la fracture.

6. Procédé selon l'une quelconque des revendications précédentes, dans lequel la mise en charge de l'emplacement de la fracture est effectuée de façon à réaliser un angle ($\vartheta$) de l'os fracturé de 2° ou moins.

7. Procédé selon l'une quelconque des revendications précédentes, dans lequel le goniomètre (10) est un électrogoniomètre comprenant une cale fine allongée (12) comportant un moyen formant jauge de contrainte (12, 13, 14) solidement fixé à au moins une de ses surfaces opposées de sorte que de l'électrogoniomètre peut être dérivé un signal représentatif de l'angle du membre fracturé lorsque l'emplacement de la fracture est mis en charge.

8. Procédé selon la revendication 7, dans lequel la cale fine allongée (12) est réalisée en acier et dans lequel le moyen formant jauge de contrainte comprend des fils métalliques (13, 13') s'étendant de manière longitudinale par rapport à la cale allongée (12).

9. Procédé selon l'une quelconque des revendications précédentes, dans lequel un signal de sortie est dérivé du goniomètre (10) qui est indicatif de l'angle de l'os sous la charge, dans lequel un signal indicatif de la force appliquée est également fourni, et dans lequel un processeur (17) reçoit lesdits signaux, et dérive une mesure de rigidité de fracture en tant que fonction de ce dernier.

10. Appareil pour la mise en oeuvre du procédé selon l'une quelconque des revendications précédentes, ledit appareil comprenant :
    un goniomètre (10) du type offrant une très petite résistance au mouvement ;
    des premier et second éléments de montage (11, 11') conçus pour supporter ledit goniomètre dans une relation coplanaire avec l'axe (9) de l'os fracturé, lesdits éléments de montage étant conçus pour être fixés par rapport à l'os fracturé de part et d'autre de l'emplacement de la fracture ;
    un moyen (16) pour mesurer la charge de l'emplacement de la fracture, produite par l'application d'une force à ce dernier dans le plan du goniomètre (10) et de l'axe de l'os (9) et dans une direction transversale à l'axe de l'os (9) ; et
    un processeur (17) pour recevoir depuis le goniomètre (10), un signal indicatif de l'angle de l'emplacement de la fracture de l'os sous la charge et pour recevoir dudit moyen de mesure de charge, un signal indicatif de la charge appliquée à l'emplacement de la fracture pour produire un tel angle, ledit processeur (17) étant conçu pour obtenir, à partir desdits signaux d'angle et de charge, une mesure de la rigidité de l'emplacement de la fracture.

8

**FIG.1**

**FIG.2**

**FIG.3**

**FIG.4**

**FIG.5**

FIG.6

FIG.7